# EUROPÄISCHE PATENTSCHRIFT

(11) **EP 1 322 265 B1**
(45) Veröffentlichungstag und Bekanntmachung des Hinweises auf die Patenterteilung: **01.03.2006**
(21) Anmeldenummer: 01960159.0
(22) Anmeldetag: 08.08.2001
(51) Int. Cl.: A61F 2/66

(54) **FUSSEINSATZ FÜR EINEN KUNSTFUSS**
FOOT INSERT FOR AN ARTIFICIAL FOOT
INSERT DE PIED POUR PIED ARTIFICIEL

(30) Priorität: 07.10.2000 DE 10049714
(43) Veröffentlichungstag der Anmeldung: 02.07.2003
(73) Patentinhaber: Otto Bock HealthCare GmbH, 37115 Duderstadt (DE)
(72) Erfinder: MOSLER, Lüder, 37115 Duderstadt (DE); PUSCH, Martin, 37115 Duderstadt (DE)
(74) Vertreter: Gramm, Werner
(86) Internationale Anmeldenummer: PCT/DE2001/003035
(87) Internationale Veröffentlichungsnummer: WO 2002/030340

(56) Entgegenhaltungen:
- EP-A- 0 648 479
- DE-A- 4 037 928
- US-A- 4 959 073
- US-A- 5 800 568
- US-A- 5 800 570
- US-A- 6 077 301

## Beschreibung

Die Erfindung betrifft einen federelastischen Fußeinsatz für einen Kunstfuß.

Ein gelenkloser Prothesenfuß mit einem die Prothesenbelastungen aufnehmenden und übertragenden federelastischen Fußeinsatz ist z.B. in der DE 40 37 928 A1 und der EP 0 648 479 A1 offenbart. Elastische Fußeinsätze zeigen auch die US-Patentschriften 4 959 073, 5 549 711 und 5 800 570, die EP 0 884 033 A2 sowie die DE 298 20 904 U1.

Der Erfindung liegt die Aufgabe zugrunde, einen federelastischen Fußeinsatz für einen Prothesenfuß zu entwickeln, der einen progressiven Knöchelmomentenverlauf aufweist, energiespeichernd wirkt und eine elastische ML(MediaI/Lateral)-Beweglichkeit zulässt.

Diese Aufgabe wird erfindungsgemäß gelöst durch einen federelastischer Fußeinsatz für einen Kunstfuß, bestehend aus zumindest zwei miteinander verbundenen Federn, die - in unbelastetem Zustand - zusammen in Seitenansicht einen etwa dreiecksförmigen Einfederraum umschließen, wobei die obere, etwa dachförmig ausgebildete Feder im Dachspitzenbereich einen Adapteranschluss und hiervon ausgehend einen konkav nach unten durchgebogenen, sich bis in den Fersenbereich erstreckenden Fersenschenkel (nachfolgend "Fersenfeder") sowie einen konkav nach unten durchgebogenen, sich bis in den Vorfußbereich erstreckenden Vorfußschenkel (nachfolgend "Vorfußfeder") aufweist, deren freien Schenkelenden mit einer separaten, den Einfederraum nach unten begrenzenden biegesteifen Basisfeder verbunden sind, die mit einer Abrollkontur so zusammenwirkt, dass beim Abrollvorgang zu Beginn der Vorfußbelastung die Kraft zunächst im Ballenbereich eingeleitet wird, wobei Vorfußfeder und Basisfeder hinsichtlich Form und Biegeelastizität so ausgelegt sind, dass sich unter Einwirkung einer zunehmenden Belastung im Vorfußbereich in diesem Bereich Vorfuß- und Basisfeder durch jeweilige Biegung sukzessive aneinander anlegen.

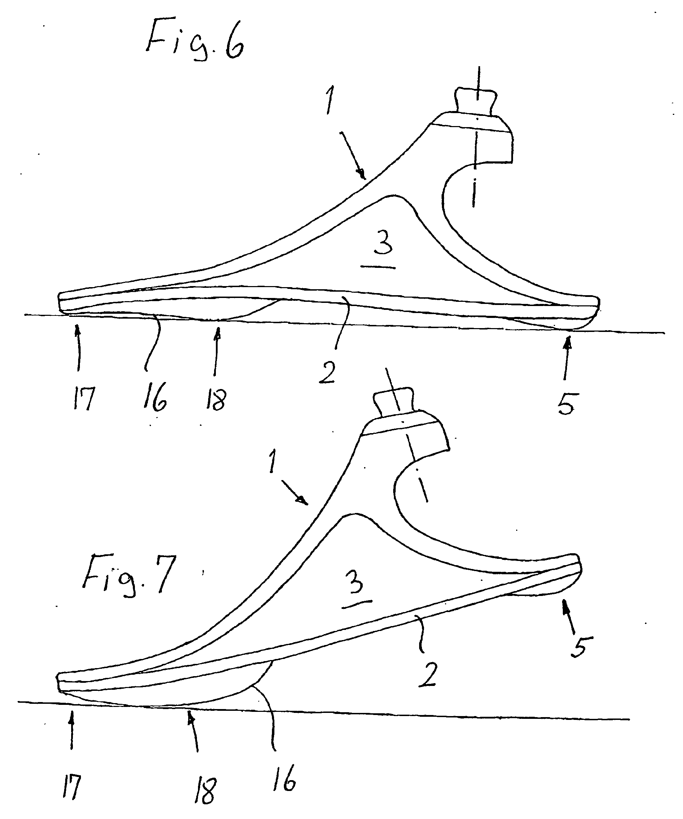

Dabei ist dieser Fußeinsatz erfindungsgemäß gekennzeichnet durch eine solche Auslegung der Vorfußfeder und Basisfeder in Form und Elastizität, dass bei zunehmender Belastung im Vorfußbereich es in diesem Bereich zu einer sukzessiven Anlage der Vorfuß- und Basisfeder aneinander kommt. Die Vorfußfeder bewirkt durch diese sukzessive Anlage an die Basisfeder einen progressiven Knöchetmomentenvertauf. Dabei wird zu Beginn der Vorfußbelastung die Kraft zunächst im Ballenbereich eingeleitet, wodurch die Basisfeder zwischen Fersen- und Vorfußfeder gelagert wird und eine Dreipunktbiegung erfährt. Es besteht dann eine Serienschaltung der Biegesteifigkeiten von Vorfuß- und Basisfeder (siehe hierzu auch die Darstellung in Figur 6). Im weiteren Verlauf des Schrittes rollt der Fuß ab und belastet zunehmend direkt die Vorfußfeder. Die Basisfeder stützt dabei zusätzlich die Momente in der Vorfußfeder ab und wird nun in umgekehrter Richtung gebogen. Es besteht eine Parallelschaltung der Biegesteifigkeiten (siehe hierzu auch die Darstellung in Figur 7). Der Ablauf der Vorfußbeias-tung ist somit von einer zunehmenden Versteifung oder Progression des Federverhaltens geprägt.

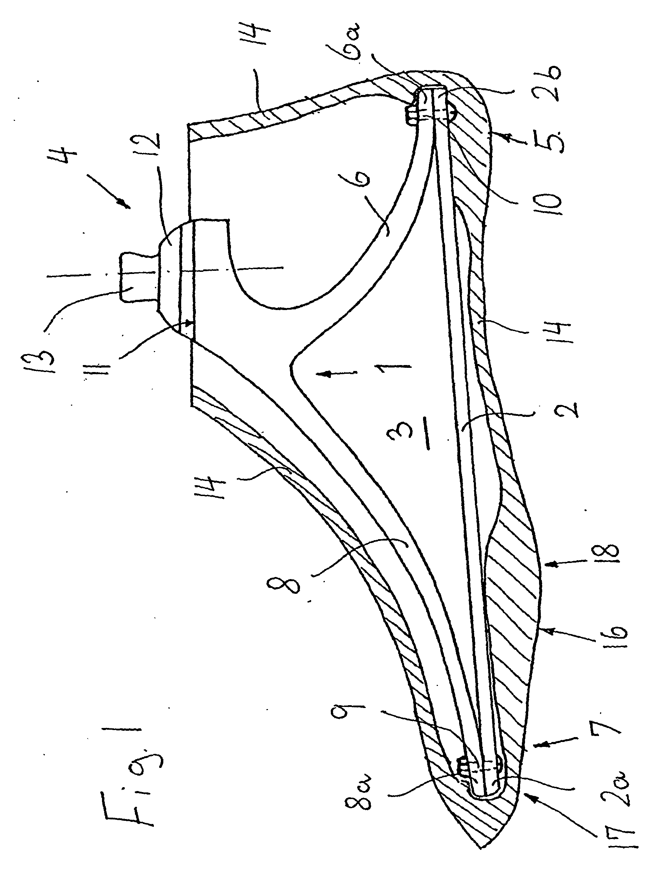

Die Kontaktpunkte des Prothesenfußes mit dem Boden oder Schuh bestimmen den Ort der Krafteinleitung. Dies hat einen Einfluss auf die Biomechanik des Fußes, steuert aber auch die Belastung der mechanischen Strukturen des Fußes. Da der Fuß während der Standphase eine Winkelbewegung erfährt, kann durch eine geeignete Abrollgeometrie der Krafteinleitungspunkt gesteuert werden. Dabei ist es unerheblich, ob diese Geometrie in die Formgebung der Basisfeder integriert ist (siehe z.B. Figur 4), auf die Basisfeder appliziert ist (siehe Figur 5) oder eine Ausprägung der umgebenden Kosmetikhülle ist (siehe Figur 1). In allen diesen Fällen wirkt die Basisfeder unterseitig mit einer Abrollkontur zusammen, die vom Zehenbereich zum Ballenbereich nach unten ansteigt, dann wieder nach oben abfällt und im Fersenbereich nach unten wulstförmig ausgeformt ist.

Es ist ferner für die angestrebte Wirkung zweckmäßig, wenn das vordere freie Schenkelende der Vorfußfeder mit dem vorderen Ende der Basisfeder verbunden ist, und wenn das hintere freie Schenkelende der Fersenfeder mit dem hinteren Ende der Basisfeder verbunden ist.

Die Charakteristik des Fußes kann gezielt verändert werden, indem Basisfedern verschiedener Steifigkeiten eingesetzt werden. Hierfür sind die Verbindungen zwischen der Basisfeder und den beiden Schenkelenden der oberen Feder lösbar gestaltet.

Durch Reduzierung der Torsionssteifigkeit der Vorfußfeder kann ML-Bewegung erzeugt werden. Dies ist dadurch realisierbar, dass die Vorfußfeder eine in ihrer Längsrichtung verlaufende längliche Ausnehmung aufweist, oder aber dadurch, dass die Vorfußfeder in ihrer Längsrichtung in zwei Teilfedern gespalten ist. Hierdurch wird auch eine Einstellung unterschiedlicher Progressionscharakteristiken für Fußinnen- und -außenseite ermöglicht. Der Momentanpol der ML-Rotation liegt in physiologisch sinnvoller Weise oberhalb der Bodenebene, während eine Rotation um die Beinlängsachse kaum möglich ist.

Zumindest eine der Federn besteht vorzugsweise aus einem Polymerwerkstoff, der zweckmäßig ein Faserverbundwerkstoff ist. Mit einem Faserverbundwerkstoff lässt sich die Krafteinleitung in die Federn dann günstiger gestalten, wenn die Federn einzeln ausgeführt werden, insbesondere dann, wenn Teile des Adapters Bestandteil einer der Federn sind. Hierfür ist es somit zweckmäßig, wenn Vorfußfeder und Fersenfeder separate Bauteile bilden, die im Bereich des Adapteranschlusses miteinander verbunden sind. Dabei ist es dann weiterhin von Vorteil, wenn zumindest die Kalotte des Adapteranschlusses integraler Bestandteil der Vorfußfeder ist. Dabei ist eine hinsichtlich der Kraftübertragung besonders günstige Konstruktion dann gegeben, wenn sich die Vorfußfeder im Bereich ihres Adapteranschlusses auf dem oberen Schenkelende der Fersenfeder abstützt.

Soll der federelastische Fußeinsatz zur Versorgung bei sehr niedrigen Amputationshöhen, insbesondere bei Exartikulationen im Sprunggelenk dienen (siehe hierzu auch die Darstellung in Figur 9), so ist es zweckmäßig, wenn der Adapteranschluss anterior des Knöchelbereiches eines natürlichen Fußes positioniert ist und eine mit der Basisfeder einen spitzen Winkel einschließende Anschlussfläche aufweist. Hierdurch kann der Raum unterhalb des Prothesenschaftes als Einfederraum für den Fersenschenkei genutzt werden. Bei dieser Befestigung sind die Momente am Adapter zwischen Fersen- und Vorfußlast ausgewogener und niedriger als bei einer Befestigung im Knöchelbereich. Somit ist die nötige Strukturfestigkeit einfacher zu erreichen.

In der Zeichnung sind einige als Beispiele dienende Ausführungsformen der Erfindung schematisch dargestellt. Es zeigen
- **Figur 1**: im Längsschnitt einen Kunstfuß bestehend aus einem federelastischen Fußeinsatz, der von einer Kosmetilchülle umgeben ist;
- **Figur 2**: in perspektivischer Darstellung einen federelastischen Fußeinsatz;
- **Figur 3**: in Draufsicht einen federelastischen Fußeinsatz;
- **Figur 4**: in Seitenansicht einen federelastischen Fußeinsatz mit einer in Form einer Abrollkontur gebogenen Basisfeder;
- **Figur 5**: in einer Darstellung gemäß Figur 4 einen elastischen Fußeinsatz mit einer geraden Basisfeder mit applizierter Abrollkontur;
- **Figur 6**: den elastischen Fußeinsatz gemäß Figur 5 zu Beginn seiner Vorfußbelastung;
- **Figur 7**: den elastischen Fußeinsatz gemäß Figur 5 mit direkt belasteter Vorfußfeder;
- **Figur 8**: einen federelastischen Fußeinsatz mit separat ausgebildeter Vorfußfeder und Fersenfeder und
- **Figur 9**: einen federelastischen Fußeinsatz zur Versorgung niedriger Amputationshöhen.

Figur 1 zeigt einen federelastischen Fußeinsatz für einen Kunstfuß. Dabei besteht der Fußeinsatz aus einer oberen, etwa dachförmig ausgebildeten Feder 1, die zusammen mit einer unteren Basisfeder 2 einen etwa dreiecksförmigen Einfederraum 3 umschließt. Die obere Feder 1 weist in ihrem Dachspitzenbereich einen Adapteranschluss 4 auf, von dem nach hinten eine konkav nach unten durchgebogene, sich bis in den Fersenbereich 5 erstreckende Fersenfeder 6 sowie nach vorn eine konkav nach unten durchgebogene, sich bis in den Vorfußbereich 7 erstreckende Vorfußfeder 8 ausgehen. Dabei ist das vordere freie Schenkelende 8a der Vorfußfeder 8 mit dem vorderen Ende 2a der Basisfeder 2 verbunden, während das hintere freie Schenkelende 6a der Fersenfeder 6 mit dem hinteren Ende 2b der Basisfeder 2 verbunden ist. Dabei können die Verbindungen zwischen der oberen Feder 1 und der Basisfeder 2 als lösbare Verbindungen 9, 10 gestaltet sein, wie es Figur 1 erkennen lässt. Die Basisfeder 2 ist dann auswechselbar.

Der Adapteranschluss 4 liegt bei den in den Figuren 1 - 8 dargestellten Ausführungsformen jeweils etwa im Knöchelbereich eines natürlichen Fußes und weist eine etwa parallel zur Basisfeder 2 liegende Anschlussfläche 11 auf. Der Adapteranschluss 4 umfasst eine Kalotte 12 und einen Pyramidenstumpf 13, der mit seiner Spitze auf der Kalotte 12 steht.

Figur 1 lässt ferner erkennen, dass der federelastische Fußeinsatz von einer Kosmetikhülle 14 umgeben ist.

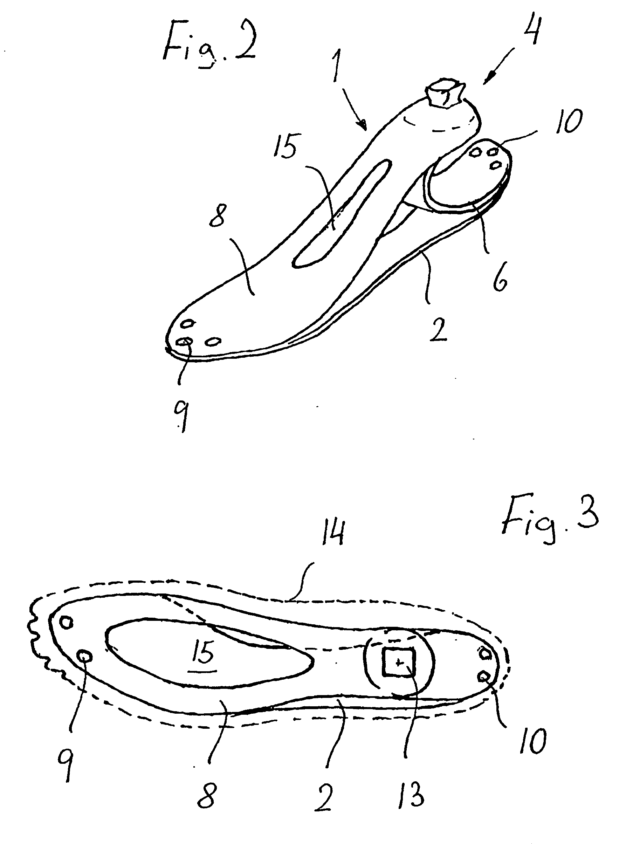

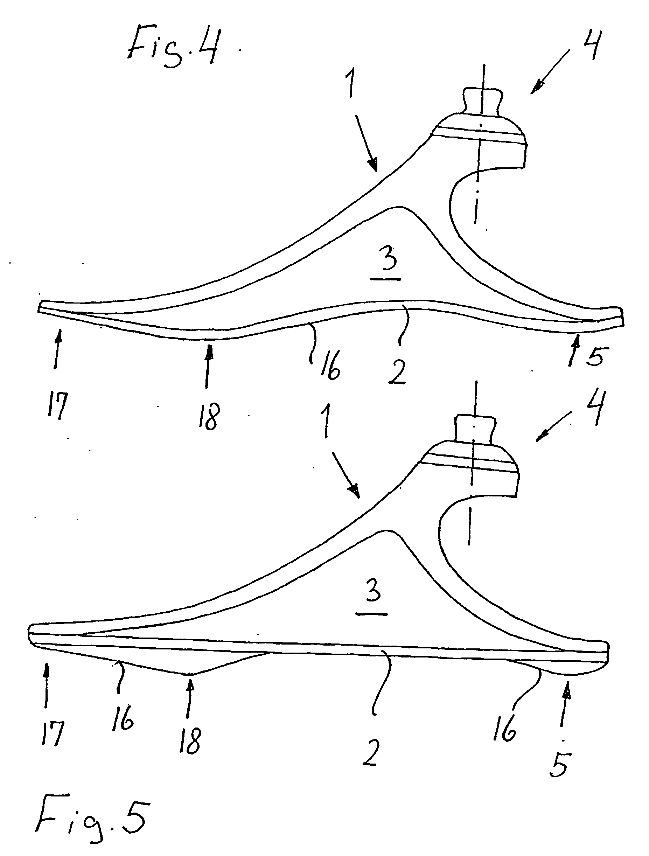

Gemäß den Figuren 2 und 3 weist die Vorfußfeder 8 eine in ihrer Längsrichtung verlaufende längliche Ausnehmung 15 auf.

Gemäß Figur 1 wirkt die im Wesentlichen gerade verlaufende Basisfeder 2 mit einer von der Unterseite der Kosmetikhülle 14 gebildeten Abrollkontur 16 zusammen, die vom Zehenbereich 17 des Kunstfußes zum Ballenbereich 18 nach unten ansteigt und dann wieder nach oben abfällt und im Fersenbereich 5 nach unten wulstförmig ausgeformt ist.

Bei der Ausführungsform gemäß Figur 4 ist die Basisfeder 2 unmittelbar in Form der Abrollkontur 16 gebogen. Bei der abgewandelten Ausführungsform gemäß Figur 5 ist die Abrollkontur 16 auf die Unterseite der in Längsrichtung etwa gerade verlaufenden Basisfeder 2 appliziert.

Bei den in den Figuren 1-7 und 9 dargestellten Ausführungsformen ist die obere Feder 1 des Fußeinsatzes einteilig ausgebildet; der Adapteranschluss 4 bildet einen integralen Bestandteil dieser einteiligen oberen Feder 1.

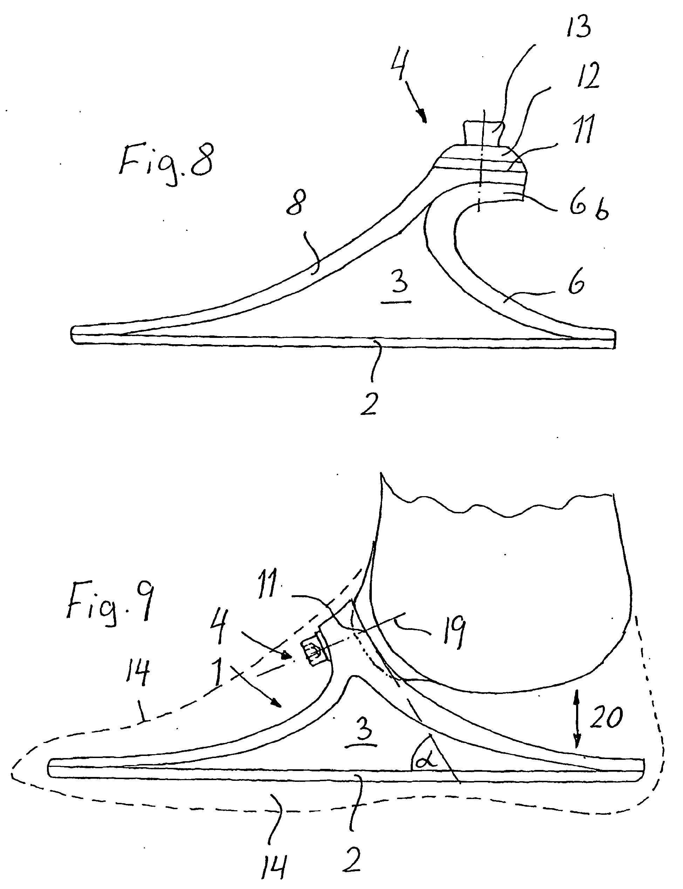

Bei der abgewandelten Ausführungsform gemäß Figur 8 bilden Fersenfeder 6 und Vorfußfeder 8 separate Bauteile, die im Bereich des Adapteranschlusses 4 miteinander verbunden sind. Dabei bildet der Adapteranschluss 4 einen integralen Bestandteil der separat ausgebildeten Vorfußfeder 8, die sich im Bereich des Adapteranschlusses 4 auf dem oberen, nach hinten umgebogenen Schenkelende 6b der Fersenfeder 6 abstützt, die angenähert C-förmig ausgebildet ist.

Figur 9 zeigt einen federelastischen Fußeinsatz, der für Versorgungen bei sehr niedrigen Amputationshöhen, insbesondere bei Exartikutationen im Sprunggelenk des Prothesenträgers bestimmt ist. Hier ist der Adapteranschluss 4 gegenüber der Ausführungsform gemäß den Figuren 1 - 8 anterior des Knöchelbereiches eines natürlichen Fußes positioniert und weist eine mit der Basisfeder 2 einen spitzen Winkel α einschließende Anschlussfläche 11 auf. Der Adapteranschluss umfasst auch hier eine Kalotte 12, die begrenzte Winkeleinstellungen des durch eine strichpunktierte Linie angedeuteten Schaftes 19 ermöglicht. Diese Ausbildung führt zu einem großen Fersenweg 20.

## Patentansprüche

1. Federelastischer Fußeinsatz für einen Kunstfuß, bestehend aus zumindest zwei miteinander verbundenen Federn (1, 2), die - in unbelastetem Zustand - zusammen in Seitenansicht einen etwa dreiecksförmigen Einfederraum (3) umschließen, **dadurch gekennzeichnet, dass** die obere, etwa dachförmig ausgebildete Feder (1) im Dachspitzenbereich einen Adapteranschluss (4) und hiervon ausgehend einen konkav nach unten durchgebogenen, sich bis in den Fersenbereich (5) erstreckenden Fersenschenkel (nachfolgend "Fersenfeder 6") sowie einen konkav nach unten durchgebogenen, sich bis in den Vorfußbereich (7) erstreckenden Vorfußschenkel (nachfolgend "Vorfußfeder 8") aufweist, deren freie Schenkelenden (6a, 8a) mit einer separaten, den Einfederraum (3) unten begrenzenden biegesteifen Basisfeder (2) verbunden sind, die mit einer Abrollkontur (16) so zusammenwirkt, dass beim Abrollvorgang zu Beginn der Vorfußbelastung die Kraft zunächst im Ballenbereich eingeleitet wird, wobei die Vorfußfeder (8) und die Basisfeder (2) hinsichtlich Form- und Biegeelastizität so ausgelegt sind, dass sich unter Einwirkung einer zunehmenden Belastung im Vorfußbereich in diesem Bereich Vorfuß- und Basisfeder (8, 2) durch die jeweilige Belastung sukzessive aneinander anlegen.

2. Fußeinsatz nach Anspruch 1, **dadurch gekennzeichnet, dass** das vordere freie Schenkelende (8a) der Vorfußfeder (8) mit dem vorderen Ende (2a) der Basisfeder (2) verbunden ist.

3. Fußeinsatz nach Anspruch 1 oder 2, **dadurch gekennzeichnet, dass** das hintere freie Schenkelende (6a) der Fersenfeder (6) mit dem hinteren Ende (2b) der Basisfeder (2) verbunden ist.

4. Fußeinsatz nach Anspruch 1, 2 oder 3, **gekennzeichnet durch** lösbar gestaltete Verbindungen (9, 10) zwischen der Basisfeder (2) und den beiden Schenkelenden (6a, 8a) der oberen Feder (1).

5. Fußeinsatz nach einem der vorhergehenden Ansprüche, **dadurch gekennzeichnet, dass** die Vorfußfeder (8) eine in ihrer Längsrichtung verlaufende längliche Ausnehmung (15) aufweist.

6. Fußeinsatz nach einem der Ansprüche 1 bis 4, **dadurch gekennzeichnet, dass** die Vorfußfeder (8) in ihrer Längsrichtung in zwei Teilfedern gespalten ist.

7. Fußeinsatz nach einem der vorhergehenden Ansprüche, **dadurch gekennzeichnet, dass** der Adapteranschluss (4) etwa im Knöchelbereich eines natürlichen Fußes positioniert ist und eine etwa parallel zur Basisfeder (2) liegende Anschlussfläche (11) aufweist.

8. Fußeinsatz nach einem der Ansprüche 1 bis 6, **dadurch gekennzeichnet, dass** der Adapteranschluss (4) anterior des Knöchelbereiches eines natürlichen Fußes positioniert ist und eine mit der Basisfeder (2) einen spitzen Winkel (α) einschließende Anschlussfläche (11) aufweist.

9. Fußeinsatz nach einem der vorhergehenden Ansprüche, **dadurch gekennzeichnet, dass** der Adapteranschluss (4) ein integraler Bestandteil der oberen Feder (1) ist.

10. Fußeinsatz nach Anspruch 9, **dadurch gekennzeichnet, dass** der Adapter-anschluss (4) eine Kalotte (12) umfasst.

11. Fußeinsatz nach Anspruch 10, **dadurch gekennzeichnet, dass** der Adapter-anschluss (4) einen Pyramidenstumpf (13) umfasst.

12. Fußeinsatz nach einem der vorhergehenden Ansprüche, **dadurch gekennzeichnet, dass** Vorfußfeder (8) und Fersenfeder (6) separate Bauteile bilden, die im Bereich des Adapteranschlusses (4) miteinander verbunden sind.

13. Fußeinsatz nach Anspruch 10 und 12, **dadurch gekennzeichnet, dass** zumindest die Kalotte (12) des Adapteranschlusses (4) integraler Bestandteil der Vorfußfeder (8) ist.

14. Fußeinsatz nach Anspruch 13, **dadurch gekennzeichnet, dass** sich die Vorfußfeder (8) im Bereich ihres Adapteranschlusses (4) auf dem oberen Schenkelende (6b) der Fersenfeder (6) abstützt.

15. Fußeinsatz nach einem der vorhergehenden Ansprüche, **dadurch gekennzeichnet, dass** zumindest eine der Federn (2, 6, 8) aus einem Polymerwerkstoff besteht.

16. Fußeinsatz nach Anspruch 15, **dadurch gekennzeichnet, dass** der Polymerwerkstoff ein Faserverbundwerkstoff ist.

17. Fußeinsatz nach einem der vorhergehenden Ansprüche, **dadurch gekennzeichnet, dass** die Basisfeder (2) unterseitig mit einer Abrollkontur (16) zusammenwirkt, die vom Zehenbereich (17) zum Ballenbereich (18) nach unten ansteigt und dann wieder nach oben abfällt.

18. Fußeinsatz nach Anspruch 17, **dadurch gekennzeichnet, dass** die Abrollkontur (16) im Fersenbereich (5) nach unten wulstförmig ausgeformt ist.

19. Fußeinsatz nach Anspruch 17 oder 18, **dadurch gekennzeichnet, dass** die Basisfeder (2) in Form der Abrollkontur (16) gebogen ist.

20. Fußeinsatz nach Anspruch 17 oder 18, **dadurch gekennzeichnet, dass** die Abrollkontur (16) auf die Unterseite der in Längsrichtung etwa gerade verlaufenden Basisfeder (2) appliziert ist.

21. Fußeinsatz nach Anspruch 17 oder 18, **dadurch gekennzeichnet, dass** die Abrollkontur (16) durch die Unterseite einer den Fußeinsatz (1, 2) umgebenden Kosmetikhülle (14) gebildet ist.

22. Fußeinsatz nach einem der vorhergehenden Ansprüche, **gekennzeichnet durch** eine solche Form der Fersenfeder (6), dass vom Fersenauftritt bis in die mittlere Standphase der Momentanpol der Rotation im Bereich des natürlichen Knöchelgelenkes liegt.

## Revendications

1. Insert de pied à élasticité ressort pour pied artificiel, comprenant au moins deux ressorts (1,2) reliés ensemble qui - dans la position de repos - vus de côté, entourent un espace intérieur aux ressorts (3) sensiblement triangulaire, **caractérisé en ce que** le ressort supérieur (1) conformé sensiblement en toit présente dans la zone de la pointe du toit un raccord d'adaptation (4) et une aile de talon (ci-après) « ressort de talon 6 ») s'étendant à partir du raccord de façon concave et courbée vers le bas jusque dans la région du talon (5) ainsi qu'une aile d'avant pied (ci-après « ressort d'avant pied 8") concave et courbée vers le bas qui s'étend jusque dans la zone avant du pied (7), dont les extrémités libres d'aile (6a, 8a) sont reliées à un ressort de base (2) rigide en flexion limitant le bas de l'espace intérieur aux ressorts (3) qui coopèrent avec un contour de roulement (16) de telle sorte que lors du processus de roulement au début de l'application de la charge sur l'avant du pied, l'effort est d'abord conduit dans la zone de la plante du pied, le ressort d'avant pied (8) et le ressort de base (2) étant agencés en ce qui concerne la forme et l'élasticité en flexion pour que sous l'effet d'une augmentation de la charge dans la zone de l'avant pied, les ressorts d'avant pied (8) et de base (2) s'appuient successivement l'un sur l'autre sous l'effet des charges appliquées respectives.

2. Insert de pied selon la revendication 1, **caractérisé en ce que** l'extrémité libre avant (8a) du ressort d'avant pied (8) est reliée à l'extrémité avant (2a) du ressort de base (2).

3. Insert de pied selon la revendication 1 ou 2, **caractérisé en ce que** l'extrémité libre arrière (6a) du ressort de talon (6) est reliée à l'extrémité libre arrière (2b) du ressort de base (2).

4. Insert de pied selon la revendication 1, 2 ou 3, **caractérisé par** des liaisons réalisées de façon amovible (9, 10) prévues entre le ressort de base (2) et les deux extrémités (6a, 8a) du ressort supérieur (1).

5. Insert de pied selon l'une des revendication précédentes, **caractérisé en ce que** le ressort d'avant pied (8) présente un évidement (15) allongé s'étendant dans la direction de sa longueur.

6. Insert de pied selon l'une des revendication 1 à 4, **caractérisé en ce que** le ressort d'avant pied (8) est séparé dans le sens de sa longueur en deux parties de ressort.

7. Insert de pied selon l'une des revendications précédentes, **caractérisé en ce que** le raccord d'adaptation (4) est positionné sensiblement dans la zone du coup de pied d'un pied naturel et présente une surface de raccord (11) située sensiblement parallèlement au ressort de base (2).

8. Insert de pied selon l'une des revendications 1 à 6, **caractérisé en ce que** le raccord d'adaptation (4) est situé en avant de la zone du coup de pied d'un pied naturel et présente une surface de raccord (11) formant un angle aigu (α) avec le ressort de base (2).

9. Insert de pied selon l'une des revendications précédentes, **caractérisé en ce que** le raccord d'adaptation (4) est un composant réalisé d'une seule pièce avec le ressort supérieur (1).

10. Insert de pied selon la revendication 9, **caractérisé en ce que** le raccord d'adaptation (4) présente une calotte (12).

11. Insert de pied selon la revendication 10, **caractérisé en ce que** le raccord d'adaptation (4) présente un embout pyramidal (13).

12. Insert de pied selon l'une des revendications précédentes, **caractérisé en ce que** le ressort d'avant pied (8) et le ressort de talon (6) sont deux pièces séparées, qui sont reliées l'une à l'autre dans la zone du raccord d'adaptation (4).

13. Insert de pied selon les revendications 10 et 12, **caractérisé en ce qu'**au moins la calotte (12) du raccord d'adaptation (4) est un composant réalisé d'une seule pièce avec le ressort d'avant pied (8).

14. Insert de pied selon la revendication 13, **caractérisé en ce que** le ressort d'avant pied (8) s'appuie dans la zone de son raccord d'adaptation (4) sur l'extrémité supérieure (6b) du ressort du talon (6).

15. Insert de pied selon l'une des revendications précédentes, **caractérisé en ce qu'**au moins l'un des ressorts (2, 6, 8) est composé d'un matériau polymère.

16. Insert de pied selon la revendication 15, **caractérisé en ce que** le matériau polymère est un matériau renforcé de fibres.

17. Insert de pied selon l'une des revendications précédentes, **caractérisé en ce que** la face inférieure du ressort de base (2) coopère avec un contour de roulement (16) qui s'étend de la zone des doigts de pied (17) jusqu'à la plante du pied (18) qui monte vers le bas et descend de nouveau vers le haut.

18. Insert de pied selon la revendication 17, **caractérisé en ce que** le contour de roulement (16) est formé dans la zone du talon (5), d'un renflement en saillie vers le bas.

19. Insert de pied selon la revendication 17 ou 18, **caractérisé en ce que** le ressort de base (2) est courbé suivant la forme du contour de roulement (16).

20. Insert de pied selon la revendication 17 ou 18, **caractérisé en ce que** le contour de roulement (16) est appliqué sur la face inférieure du ressort de base (2) s'étendant de façon sensiblement rectiligne dans le sens de la longueur.

21. Insert de pied selon la revendication 17 ou 18, **caractérisé en ce que** le contour de roulement (16) est réalisé sur la face inférieure d'une enveloppe cosmétique (14) entourant l'insert de pied (1, 2).

22. Insert de pied selon l'une des revendications précédentes, **caractérisé par** une forme du ressort de talon (6) telle que le centre de rotation instantané se trouve dans la zone de l'articulation naturelle du coup de pied, entre l'appui sur le talon jusque dans la phase centrale de la position debout.

## Claims

1. Resilient foot insert for an artificial foot, consisting of at least two springs (1, 2) which are connected to one another and which together in side view, in the unloaded state, enclose an approximately triangular spring deflection space (3), **characterized in that** the upper, approximately roof-shaped spring (1) has, in the roof top area, an adapter attachment (4) and, starting from the latter, a heel branch (hereinafter "heel spring 6") which extends downward in a concave curve into the heel area (5), and a forefoot branch (hereinafter "forefoot spring 8") which extends downward in a concave curve into the forefoot area (7), the free branch ends (6a, 8a) being connected to a separate, flexurally rigid base spring (2) which delimits the bottom of the spring deflection space (3) and which interacts with a rolling contour (16) in such a way that, at the start of loading of the forefoot during the rolling action, the force is first introduced in the area of the ball of the foot, with the forefoot spring (8) and the base spring (2) being configured in terms of shape and flexural elasticity in such a way that, under the effect of an increasing load in the forefoot area, the forefoot spring (8) and the base spring (2) successively bear against one another in this area as a result of the respective load.

2. Foot insert according to Claim 1, **characterized in that** the front free branch end (8a) of the forefoot spring (8) is connected to the front end (2a) of the base spring (2) .

3. Foot insert according to Claim 1 or 2, **characterized in that** the rear free branch end (6a) of the heel spring (6) is connected to the rear end (2b) of the base spring (2).

4. Foot insert according to Claim 1, 2 or 3, **characterized by** releasable connections (9, 10) between the base spring (2) and the two branch ends (6a, 8a) of the upper spring (1).

5. Foot insert according to one of the preceding claims, **characterized in that** the forefoot spring (8) has an oblong recess (15) extending in its longitudinal direction.

6. Foot insert according to one of Claims 1 to 4, **characterized in that** the forefoot spring (8) is split into two part-springs in its longitudinal direction.

7. Foot insert according to one of the preceding claims, **characterized in that** the adapter attachment (4) is positioned approximately in the ankle area of a natural foot and has an attachment surface (11) lying approximately parallel to the base spring (2) .

8. Foot insert according to one of Claims 1 to 6, **characterized in that** the adapter attachment (4) is positioned anteriorly of the ankle area of a natural foot and has an attachment surface (11) enclosing an acute angle (α) with the base spring (2).

9. Foot insert according to one of the preceding claims, **characterized in that** the adapter attachment (4) is an integral component part of the upper spring (1).

10. Foot insert according to Claim 9, **characterized in that** the adapter attachment (4) comprises a spherical cap (12).

11. Foot insert according to Claim 10, **characterized in that** the adapter attachment (4) comprises a truncated pyramid (13).

12. Foot insert according to one of the preceding claims, **characterized in that** the forefoot spring (8) and heel spring (6) form separate structural parts, which are connected to one another in the area of the adapter attachment (4).

13. Foot insert according to Claim 10 and Claim 12, **characterized in that** at least the spherical cap (12) of the adapter attachment (4) is an integral component part of the forefoot spring (8).

14. Foot insert according to Claim 13, **characterized in that** the forefoot spring (8), in the area of its adapter attachment (4), is supported on the upper branch end (6b) of the heel spring (6).

15. Foot insert according to one of the preceding claims, **characterized in that** at least one of the springs (2, 6, 8) is made of a polymer material.

16. Foot insert according to Claim 15, **characterized in that** the polymer material is a fibre composite.

17. Foot insert according to one of the preceding claims, **characterized in that** the base spring (2) cooperates on the underside with a rolling contour (16) which slopes downwards from the toe area (17) to the ball area (18) and then slopes upward again.

18. Foot insert according to Claim 17, **characterized in that** the rolling contour (16) is shaped as a downward bulge formation in the heel area (5).

19. Foot insert according to Claim 17 or 18, **characterized in that** the base spring (2) is bent in the shape of the rolling contour (16).

20. Foot insert according to Claim 17 or 18, **characterized in that** the rolling contour (16) is applied to the underside of the base spring (2) extending approximately in a straight line in the longitudinal direction.

21. Foot insert according to Claim 17 or 18, **characterized in that** the rolling contour (16) is formed by the underside of a cosmetic covering (14) which surrounds the foot insert (1, 2).

22. Foot insert according to one of the preceding claims, **characterized by** the heel spring (6) having such a shape that, from the heel touching the ground to the middle of the stance phase, the instantaneous centre of rotation lies in the area of the natural ankle joint.
